(19) Europäisches Patentamt
European Patent Office
Office européen des brevets

(11) Publication number: **0 033 537**
**B1**

(12) # EUROPEAN PATENT SPECIFICATION

(45) Date of publication of patent specification: **29.05.85**

(51) Int. Cl.[4]: **C 07 D 309/30,** C 07 C 69/30, A 61 K 31/22, A 61 K 31/365

(21) Application number: **81100728.5**

(22) Date of filing: **02.02.81**

(54) **Hydrogenation products of mevinolin and dihydromevinolin, a process for preparing the same and an antihypercholesterolemic pharmaceutical composition containing the same.**

(30) Priority: **04.02.80 US 118050**

(43) Date of publication of application:
**12.08.81 Bulletin 81/32**

(45) Publication of the grant of the patent:
**29.05.85 Bulletin 85/22**

(84) Designated Contracting States:
**AT BE CH DE FR GB IT LI LU NL SE**

(56) References cited:
**EP-A-0 022 478**
**DE-A-3 006 216**
**US-A-3 983 140**
**US-A-4 049 495**
**US-A-4 231 938**

**Patents Abstracts of Japan Vol. 4, No. 34, 22 March 1980 page 111C3**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Patchett, Arthur A.**
**1090 Minisink Way**
**Westfield New Jersey 07090 (US)**
Inventor: **Kuo, Chan-Hwa**
**105 East Nassau Avenue**
**South Plainfield New Jersey 07080 (US)**

(74) Representative: **Abitz, Walter, Dr.-Ing. et al**
**Abitz, Morf, Gritschneder P.O. Box 86 01 09**
**D-8000 München 86 (DE)**

EP 0 033 537 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Summary of the Invention

This invention relates to hypocholesterolemic products produced by hydrogenation of a compound of formula I and a naturally occurring dihydro analog thereof.

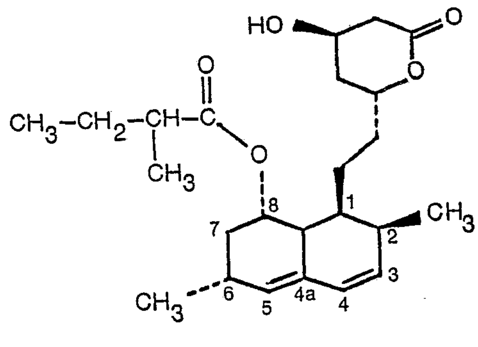

I

More specifically, it relates to compounds of the formula II and to the corresponding free hydroxy acid of formula III and to processes for their preparation. Further, it relates to the pharmaceutical acceptable salts of free acid III and to the methyl, ethyl, propyl, butyl, isobutyl, amyl, isoamyl, 2-dimethylaminoethyl, benzyl, phenethyl and 2-acetamidoethyl esters thereof.

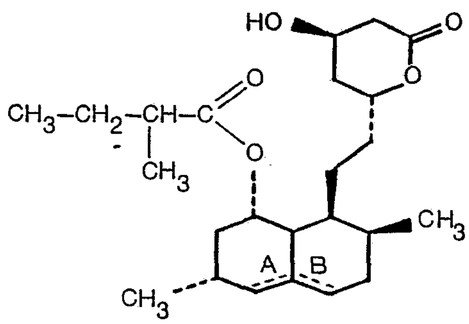

II

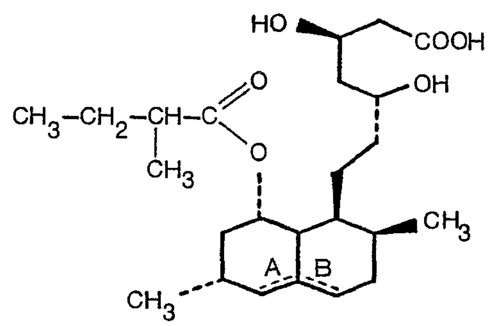

III

The dotted lines A and B represent optional double bonds not more than one of A and B being double bond. These new compounds have excellent propeties of inhibiting cholesterol biosynthesis and are useful against hypercholesterolemia and hyperlipemia.

Background of the Invention

Because of the possible connection between high blood cholesterol and atherosclerosis, many efforts have been made to find ways and substances which would reduce the cholesterol in the mammalian body. One of these ways is to inhibit in mammals the body's ability to synthesize cholesterol.

Recently, Endo et al., described (JP—A—55—9024, U.S. 4,049,495 and 3,983,140) a fermentation product obtained by cultivation of a microorganism of the genus *Penicillium* and isolation from the medium. They called it ML236N and determined its structure together with two related compounds 236A and 236C. Its structure, under the name compactin, was also determined by A. G. Brown, T. C. Smale, T. J. King, *J. Chem. Soc.* (Perkin I) 1165(1975). This compound has been found to be a strong inhibitor in vivo of the biosynthesis of cholesterol.

More recently, Monaghan et al. have reported (U.S. Patent 4,231,938) that a methyl analog (Formula I) is formed by the cultivation of a microfungus of the genus *Aspergillus* and that this product is an even more potent inhibitor of cholesterol biosynthesis.

From the same fermentation, Albers-Schonberg et. al. EP—A1—0022478 isolated the dihydro analog of structure IV.

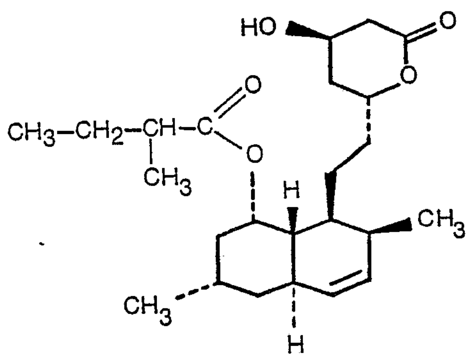

IV

Also more recently, Endo has reported (J. Antibiotics, August 1979, page 852, and W. German Offenlegungschrift 3006216), the isolation of a compound identical with Compound I from a cultivation of a strain of *Monascus ruber*, an entirely different microorganism.

Description of the Invention

We have found that the hydrogenation of Compounds I and IV over various catalysts produces dihydro (from I) and tetrahydro products, some of which (V, VI and VIII as shown in Flow Sheet A) are potent inhibitors of cholesterol synthesis, while others (e.g. VII) are not.

These new compounds do not appear to be formed in the fermentations described by Endo or Monaghan or Albers-Schonberg. They are stable compounds even in thin films and are potent inhibitors of cholesterol synthesis equal or superior to the compound, ML236B described by Endo in this respect.

The compounds of this invention are highly useful as antihypercholesterolemic agents for the treatment of atherosclerosis, hyperlipemia and like diseases in animals and humans. They may be administered orally or parenterally in the form of a capsule, a tablet, an injectable preparation or the like. It is usually desirable to use the oral route. Doses may be varied, depending on the age, severity, body weight and other conditions of animal or human patients but daily dosage for human adults is within a range of from about 2 mg. to 2000 mg. (preferably 10 to 100 mg.) given in three or four divided doses. Higher doses may be favourably applied as required.

The compounds of this invention also have useful anti-fungal activities. For example, they may be used to control strains of *Penicillium sp., Aspergillus niger, Cladosporium sp., Cochliobolus miyabeanus* and *Helminthosporium cynodnotis*. For those utilities they are admixed with suitable formulating agents, powders, emulsifying agents or solvents such as aqueous ethanol and sprayed or dusted on the items to be protected.

The preparation of the compounds of this invention is described in Flow Sheet A. One starting material is the fermentation product, Compound I, described by Monaghan et al, U.S. Patent 4,231,938. This, depending on the catalyst used, can be hydrogenated to give one of the compounds V or VI or a mixture of compounds VII and VIII. Thus, hydrogenation over tris(triphenylphosphine)chlororhodium in toluene, saturates the 3,4 double bond to give compound VI but hydrogenation over palladium on calcium carbonate, in ethanol not only saturates the 4a, 5 double bond but causes a shift of the 3, 4 double bond to the 4, 4a position (i.e. there is effectively a 1, 4 hydrogenation of the conjugated diene). Hydrogenation over platinum oxide in ethyl acetate completely saturates the polyhydronaphthyl ring to give a mixture of the two isomeric decalin derivatives VII and VIII. The naturally occurring dihydro Compound I, i.e. Compound IV described by Albers-Schonberg et al. (European Patent Application 80,103,286), on reduction over palladium on charcoal in ethyl acetate also provides the completely saturated polyhydronaphthalene ring to give only the compound of Structure VIII with the trans ring fusion.

FLOW SHEET A

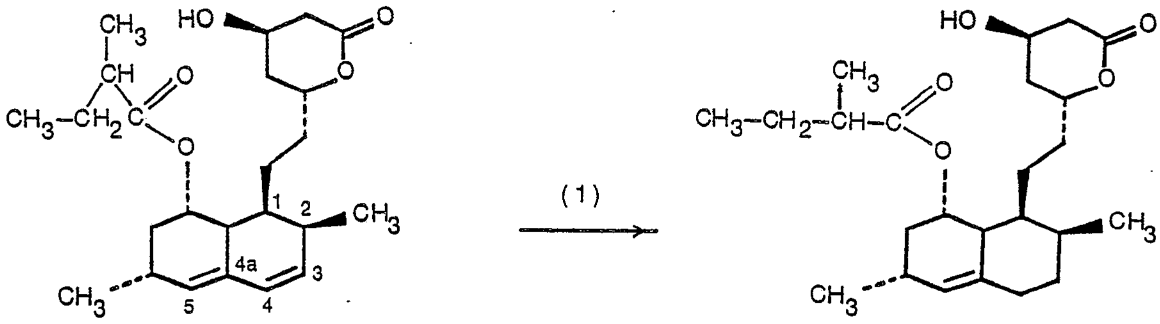


I VI

I
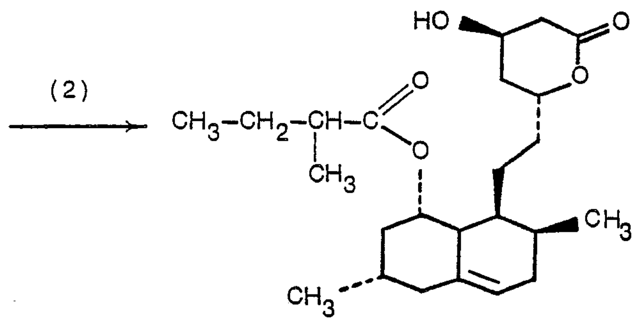


V

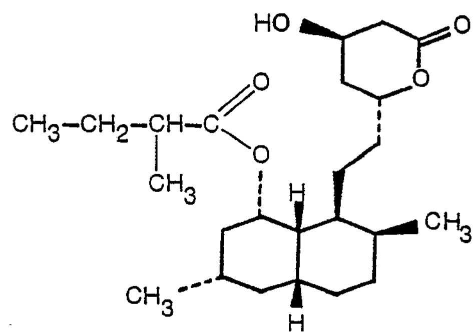

VII

I
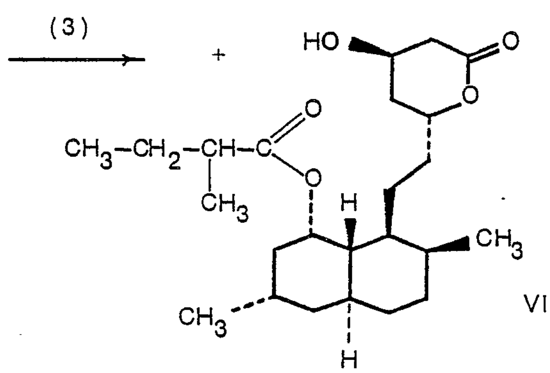


VIII

IV (4) VIII

Reactions and Reagents

1. Hydrogenation at about 20—75°C and about atmospheric pressure to about 4 atmospheres over tris (triphenylphosphine) chlororhodium in an aromatic solvent such as benzene, toluene or xylene, preferably toluene. Preferred conditions are about 40°C and about 2—7 atmospheres in toluene.

2. Hydrogenation at about 20—25°C and about atmospheric pressure over 5% palladium on calcium carbonate in a lower alkanol such as $C_{1-3}$ alkanol, especially ethanol.

3. Hydrogenation at about 20—25°C and atmospheric pressure over platinum oxide in ethyl acetate.

4. Hydrogenation at 20—25°C and atmospheric pressure over 10% Palladium on charcoal in ethyl acetate.

Example 1

6α[2-(8β-2-S-methylbutyryloxy-2β, 6α-dimethyl-1,2,3,4,6,7,8,8a-octahydronaphthyl-1)ethyl]-4β-hydroxy-3,4,5,6-tetrahydro-2H-pyran-2-one. (VI)

Step A: Preparation of Compound I

1. *Fermentation*

A tube of lyophilized culture MF-4845 *Aspergillus terreus*, ATCC No. 20542, American Type Culture Collection, Rockville, Maryland, USA, was opened aseptically and the content suspended in an unbaffled 250 ml Erlenmeyer flask (seed flask) containing approximately 10 ml of the Medium which has the following composition:

| | |
|---|---|
| Medium | |
| Corn steep liquor | 5 g |
| Tomato paste | 40 g |
| Oatmeal | 10 g |
| Glucose | 10 g |
| Trace Element Solution | 10 g |
| Distilled water | 1000 ml |
| pH 6.8 with NaOH | |
| Trace Element Solution: | |
| $FeSO_4.7H_2O$ | 1000 mg |
| $MnSO_4.4H_2O$ | 1000 mg |
| $CuCl_2.2H_2O$ | 25 mg |
| $CaCl_2.2H_2O$ | 100 mg |
| $H_3BO_3$ | 56 mg |
| $(NH_4)_6Mo_7O_{24}.4H_2O$ | 19 mg |
| $ZnSO_4.7H_2O$ | 200 mg |
| Distilled Deionized Water | 1000 ml |

The inoculated flask was incubated for 24 hours at 28°C on a 220 rpm shaker (5.08 cm, 2 inch throw). An unbaffled 2 liter Erlenmeyer flask containing 500 ml of the medium was then inoculated with 10 ml of the first stage fermentation growth from the seed mixture. This too was shaken 24 hours at 28°C.

A 757 l, (200 gallon) stainless steel fermentation vat was then charged with 485 liters of a medium comprising:

| | |
|---|---|
| Cerelose | 4.5% wt/vol |
| Peptonized Milk | 2.5% wt/vol |
| Autolyzed yeast | 0.25% wt/vol |
| Polyglycol P2000 | 0.25% vol/vol |

whose pH was adjusted to 7.0. This was sterilized 15 minutes at 121°C. One liter of the second stage above was then charged and the mixture was incubated at 85 rpm for 12 hours then 130 rpm for 84 hours at 28°C with an air flow of 5 cfm for 12 hours then 10 cfm for 84 hours.

2. *Isolation*

a. *Extraction*

Two batches of 378 l (one hundred gallons) of whole broth were combined, acidified with stirring to pH 4.1 by careful addition of 800 ml of concentrated hydrochloric acid, and extracted by addition of 283 l (75 gal) of ethyl acetate and further stirring for two hours.

About 11.3 kgs (25 lbs) of a silicaceous filter aid was then added and the total slurry was pumped through a 24-inch filter press. An additional 283 l (75 gal) of ethyl acetate was used to wash the press cake and continue the extraction, by reversing the direction of pumping through the press four times. Then all of the wash solvent was discharged from the press and combined with the first filtrate. The two-phase filtrate was allowed to settle, and the water layer removed. The ethyl acetate layer was washed with 37.8 l (10 gal) of deionized water, the phases were allowed to separate and the ethyl acetate extracts were concentrated under vacuum to a residue of about 37.8 l (10 gal).

b. *Lactonization*

Ethyl acetate extracts from an additional 1135 l (three hundred gal) of broth were added to the above extract and the volume was reduced to about 113.5 l (thirty gal.) by vacuum distillation. About 189 l (fifty gal.) of toluene was added, and the batch was concentrated under vacuum to 121 l (32 gal.); this step was repeated; then sufficient new toluene was added to bring the volume to 283 l (75 gal.). Without vacuum, the batch was brought to reflux and maintained there for two hours, with a temperature over 106°C.

This solution was then concentrated under vacuum to a small volume, which was further concentrated to an oily residue in a large rotary evaporator under vacuum.

c. *Chromatography on Silica Gel*

The extract obtained above was flushed free of other solvents by addition of 7.6 l (2 gal.) of methylene chloride and reconcentration to an oil.

The oily residue was dissolved in about 18.9 l (5 gal.) of ethyl acetate-methylene chloride (30/70; v/v) mixture, and a slurry was made by addition of 2.8 kg of silica gel.

The slurry was loaded as a level layer on the top of a 30,5 × 127 cm (12 in. × 50 in.) silica gel column packed in the same solvent mixture.

Elution was with ethyl acetate-methylene chloride (40/60; v/v) at 800 ml/min. A forerun of 37.9 l (10 gal.) then further fractions of 15 l (4 gal). each were collected.

Fractions 6—10 inclusive were concentrated under vacuum to an oily residue which was dissolved in hot ethyl acetate, treated with decolorizing carbon, filtered hot, and cooled. Crystals of Compound I were filtered off and the mother liquors were concentrated to an oil. The crystalline material (I) after recrystallization from ethanol has m.p. 170—171°C.

Step B: Preparation of 6α[2-(8β-2-S-methylbutyryloxy-2β,6α-dimethyl-1,2,3,4,6,7,8,8a-octahydronaphthyl-1)ethyl]-4β-hydroxy-3,4,5,6-tetrahydro-2H-pyran-2-one. (VI)

A mixture of 50 mg (0.1236 mmol) of Compound I and an equal molar amount (114.35 mg., 0.1236 mmol) of tris-(triphenylphosphine)-chlororhodium in 10 ml of dry toluene was hydrogenated at room temperature for 6 days, with a total uptake of 14.6 ml of hydrogen. The mixture was evaporated *in vacuo* to dryness. The red filtrate was subjected to preparative thin layer chromatography on silver nitrate impregnated silica plates and was developed twice in 10% ethyl acetate-ether system. The yield of compound VI was 22.3 mg.

Mass spectra (M/e) 406 ($m^+$)
304 (m-102)
286 (m-102-18)

nmr ($CDCl_3$, 300 MHz)
δ 4.37 (m, 1H)
4.60 (m, 1H)
5.34 (d of t, J = 2.5 Hz, 1H)
5.41 (m, 1H)

Example 2

6α[2-(8β-2-S-methylbutyryloxy-2β, 6α - dimethyl - 1,2,3,4,6,7,8,8a - octahydronaphthyl - 1)ethyl] - 4β - hydroxy - 3,4,5,6 - tetrahydro - 2H - Pyran - 2 - one. (VI)

A solution of 1.21 g (3 mmol) of Compound I in 80 cc toluene was added to 1.21 g of tris-(triphenylphosphine)-chlororhodium prereduced in 20 cc of toluene and hydrogenated at 40° and 2.65 bar (2.7 atmospheres) of hydrogen pressure for 24 hours. The reaction mixture was concentrated *in vacuo*, taken up in 50 cc of ether, treated with decolorizing carbon and filtered through a diatomaceous filter aid to give, after concentration, an oil, wt. 2.3 g. This product was chromatographed by dry column technique on 250 g of silica gel and eluted with 30% acetone-hexane. From this chromatography were obtained single spot fractions ($R_f$ 0.48) totalling 650 mg. This product was transparent in the uv and gave a mass ion of 406. *Anal.* Calcd. for $C_{24}H_{38}O_5$: C, 70.90; H, 9.42. Found: C, 71.17; H, 9.41. The $C^{13}$ nmr of this product demonstrated it to be an approximately 9:1 mixture of the desired Compound VI together with the double bond isomer V. Crystallization from acetone-hexane gave, after several crystallizations, Compound VI (needles) of constant melting point, 113—115°.

Example 3

6α[2 - (8β - 2 - (S) - methylbutyryloxy - 2β,6α - dimethyl - 1,2,3,5,6,7,8,8a - octahydronaphthyl - 1)ethyl] - 4β - hydroxy - 3,4,5,6 - tetrahydro - 2H - pyran - 2 - one. (V).

A solution of 80.91 mg (0.2 mmol) of Compound I in 10 ml of absolute ethanol, in the presence of an equal weight of 5% Pd on $CaCO_3$ was hydrogenated at 1 atmosphere until an uptake of one mole equivalent of hydrogen was observed. The catalyst was then removed by filtration and the filtrate was evaporated to dryness (81 mg). After a purification by preparative thin layer chromatography to remove a small amount of by-product dihydro compound VI, 72 mg. of the 1,4 reduction product (V) was isolated.

Mass spectra (M/e)
406 ($m^+$)
304 (m-102)
286 (304-$H_2O$)

Nmr ($CDCl_3$, 300 MHz)
δ 4.38 (m, 1H)
4.64 (m, 1H)
5.28 (d of t, J = 3.5 Hz, 1H)
5.48 (m, 1H)

Example 4

6α - [2 - (8β - 2(S) - methylbutyryloxy - 2α,6β - dimethyl - 1,2,3,4,4aα,5,6,7,8,8a - decahydronaphthyl - 1) - ethyl] - 4β - hydroxy - 3,4,5,6 - tetrahydro - 2H - pyran - 2 - one, (VIII)

A solution of 80.91 mg (0.2 mmol) of Compound I in 10 ml of ethyl acetate was hydrogenated in the presence of an equal weight of platinum oxide at one atmosphere. An exact 2 mole equivalent of hydrogen was consumed within 1 hour. The catalyst was removed by filtration and the filtrate was concentrated to dryness to give an oil. The cis and trans isomers were separated by preparative thin layer chromatography on silica gel plates (10% ethyl acetate — ether system, bands detected by water spray). The trans isomer (VIII) appears as a more polar spot, compared to the cis isomer, and 60 mg was isolated.

Mass spectra (M/e) 408 ($m^+$)
323 (m-85)
306 (m-102)

nmr ($CDCl_3$, 300 MHz)
δ 4.36 (broad singlet, 1H)
4.59 (m, 1H)
5.19 (d of t, J = 2.5 Hz, 1H)

Example 5

6α - [2 - (8β - 2(S) - methylbutyryloxy - 2α,6β - dimethyl - 1,2,3,4,4aα,5,6,7,8,8a - decahydronaphthyl - 1)ethyl] - 4β - hydroxy - 3,4,5,6 - tetrahydro - 2H - pyran - 2 - one (VIII)

Step A: Isolation of Compound IV

1. *Rechromatography on Silica Gel*

Mother liquor residues from broth extract work-ups equivalent to an additional 2271 l (600 gal.) of fermentation production as described in Example 1, Step A were combined with the concentrated mother liquors from Example 1, Step A, in methylene chloride solution. One-half of this solution was taken for further silica gel chromatography. A small aliquot showed a total solids content of 325 g. The solution was treated with 40 g of decolorizing carbon, filtered, and the cake rinsed with methylene chloride. The combined filtrate and washings were concentrated under vacuum to an oily residue. This was redissolved in 800 ml of ethyl acetate/methylene chloride (30/70; v/v) and slurried with 225 g of silica gel. The slurry was loaded on top of a 14 × 36 cm column bed of silica gel packed in the same solvent mixture. Development

was with ethyl acetate/methylene chloride (40/60; v/v). A forecut of three liters was set aside; then fractions of 800 ml each were collected.

2. *Chromatography on Reverse-phase Packing*

Forty ml from fraction 12 of the above chromatography were concentrated to an oil weighing 500 mg and the oil redissolved in 5 ml acetonitrile. This acetonitrile solution was charged to a 1.59 cm (5/8 ") OD by 1.8 m (6 ft) long stainless steel chromatography column packed with preparative reverse-phase liquid chromatography column packing material "Bondapak C18/PorasilB" (Trademark, Waters Associates, Inc., Milford, Mass. 01757). The column was eluted with a mixture consisting of (v/v) 55% acetonitrile and 45% of 0.05 M ammonium phosphate pH 3. The elution volume between 1360 ml and 1700 ml was combined on the basis of refractive index detection. The organic solvent was removed *in vacuo* and the residual aqueous solution extracted with ethyl acetate. *In vacuo* removal of the ethyl acetate left 120 mg of compound which crystallized from a concentrated acetonitrile solution yielding crystals of Compound IV, m.p. 129—131°C.

Step B: Preparation of 6α - [2 - (8β - 2(S) - methylbutyryloxy - 2α,6β - dimethyl - 1,2,3,4,4aα,5,6,7,8,8a - decahydro - naphthyl - 1) - ethyl] - 4β - hydroxy - 3,4,5,6 - tetrahydro - 2H - pyran - 2 - one (VIII)

A solution of 90 mg Compound IV, from Step A, in 5 cc ethyl acetate and 90 mg of 10% Pd/charcoal was hydrogenated at 24° and atmospheric pressure. Hydrogen absorption was immediate and complete. The reaction mixture was filtered through a diatemaceous filter aid, concentrated *in vacuo* and crystallized from hexane-acetone, m.p. 126—127°. It was single spot on tlc (acetone-hexane 30:70), $R_f$ 0.4.

Example 6

Salts

To a solution of 40 mg of the product of Example 1 in 2 ml of ethanol is added 1 ml of aqueous NaOH ($10^{-4}$ moles; 1 equivalent). After one hour at room temperature, the mixture is taken to dryness *in vacuo* to yield the sodium salt of the free acid form of Compound VI.

In like manner the potassium salt is prepared using one equivalent of potassium hydroxide, the calcium salt using CaO, and the ammonium salt using ammonium hydroxide. Other pharmaceutically acceptable salts are likewise prepared using equivalent quantities of the appropriate base.

In like manner, the products of Examples 3, 4 and 5 can be converted into salts.

Example 7

Preparation of Free Hydroxy Acids

The sodium salt produced in Example 6, from the product of Example 1 is redissolved in 2 ml of ethanol-water (1:1) and added to 10 ml of 1N hydrochloric acid from which the liberated hydroxy acid is extracted with ethyl acetate. The latter solvent is washed once with water, dried and removed *in vacuo* with a bath temperature not exceeding 30°. The hydroxy acid derived slowly reverts to the lactone on standing.

In like manner, the sodium salts of the products of Example 3, 4 and 5 are converted to the free hydroxy acids.

Example 8

To a solution of 4 mg of the product of Example 1 in 1 ml of absolute ethanol is added 0.1 ml 0.1 M sodium ethoxide in absolute ethanol. This solution is allowed to stand at room temperature for one hour, is then diluted into water and extracted twice with ethyl acetate, the ethyl acetate, dried over anhydrous sodium sulfate, is removed *in vacuo* to yield the ethyl ester of the hydroxy acid of compound VI.

In like manner, by the use of equivalent amounts of methanol, propanol, butanol, isobutanol, amylalcohol, isoamylalcohol, 2-dimethylaminoethanol, benzylalcohol, phenethanol or 2-acetamido-ethanol, the corresponding esters are obtained.

In like manner, using the Compounds V and VIII prepared in Example 3 and 4 and 5 in the above procedures, the corresponding esters are prepared.

**Claims for the Contracting States: BE CH DE FR GB IT LI LU NL SE**

1. A compound of the formula

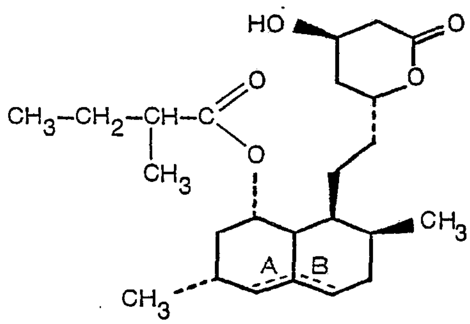

II

and its free acid form

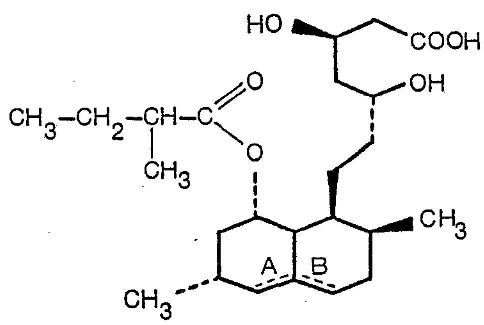

III

in which the dotted lines indicated as A and B represent optional double bonds, not more than one of A and B being a double bond, and, where neither A nor B is a double bond, the perhydronaphthyl ring is in the 4aα, 8aβ configuration, together with the pharmaceutically acceptable salts of said free acid and the methyl, ethyl, propyl, butyl, isobutyl, amyl, isoamyl, 2-dimethylaminoethyl, benzyl, phenethyl, and 2-acetamidoethyl esters.

2. The compound of Claim 1 which is the lactone of Formula II.

3. The pharmaceutically acceptable salts of the free acid of Formula III of Claim 1.

4. The free acid form of Compound III of Claim 1.

5. The compounds of Claim 2 in which A is a double bond.

6. The compound of Claim 2 in which B is a double bond.

7. The compounds of Claim 2 in which neither A nor B is a double bond and the ring system is transfused.

8. A process of producing the compounds of Claim 1 which comprises subjecting the compound

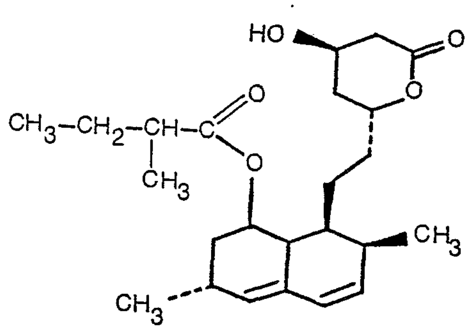

to hydrogenation either

a) over tris (triphenylphosphonium)rhodium chloride to produce a compound in which B is a double bond, or

b) over palladium on calcium carbonate to produce a compound in which A is a double bond, or

c) over platinum oxide to produce a compound in which neither A nor B is a double bond.

9. A process of producing the compound of Claim 1, wherein neither A nor B is a double bond which comprises subjecting the compound

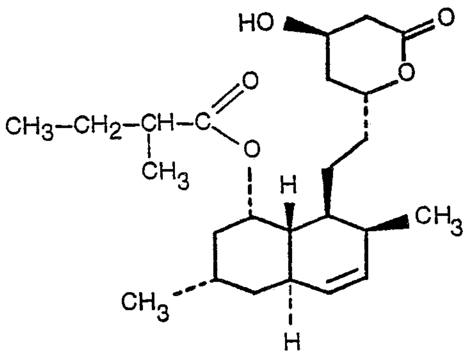

IV

to hydrogenation over palladium on charcoal.

10. An antihypercholesterolemic pharmaceutical composition comprising a pharmaceutical carrier and an effective amount of a compound of formula II or III of claim 1.

**Claims for the Contracting State: AT**

1. A process of producing a compound of the formula:

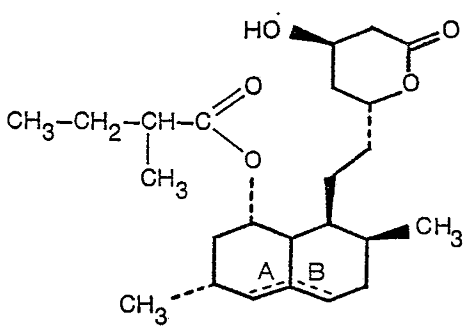

II

and its free acid form

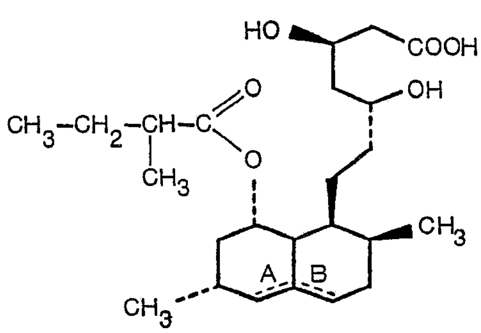

III

in which the dotted lines indicated as A and B represent optional double bonds, not more than one of A and B being a double bond, and, where neither A nor B is a double bond, the perhydronaphthyl ring is in the 4aα, 8aβ configuration, together with the pharmaceutically acceptable salts of said free acid, and the methyl, ethyl, propyl, butyl, isobutyl, amyl, isoamyl, 2-dimethylaminoethyl, benzyl, phenethyl, and 2-acetamidoethyl esters, which comprises subjecting the compound

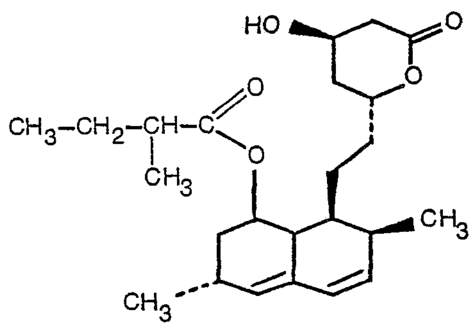

to hydrogenation either

a) over tris (triphenylphosphonium)rhodium chloride to produce a compound in which B is a double bond, or

b) over palladium on calcium carbonate to produce a compound in which A is a double bond, or

c) over platinum oxide to produce a compound in which neither A nor B is a double bond, and, if desired, reacting the resulting pyranone compound with an appropriate base and, if desired, reacting the resulting salt of the acid compound with an anorganic acid, or, if desired, reacting the resulting pyranone compound with the alkoxide corresponding to the above-mentioned esters.

2. A process of producing the compound of Claim 1, wherein neither A nor B is a double bond which comprises subjecting the compound

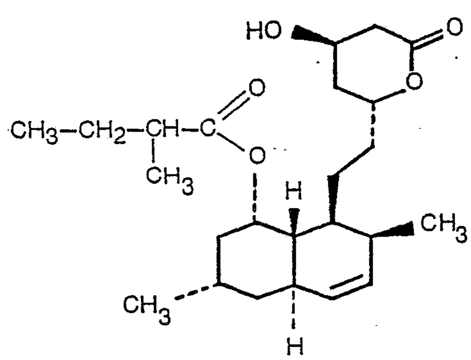

IV

to hydrogenation over palladium on charcoal.

**Patentansprüche für die Vertragsstaaten: BE CH DE FR GB IT LI LU NL SE**

1. Eine Verbindung der Formel:

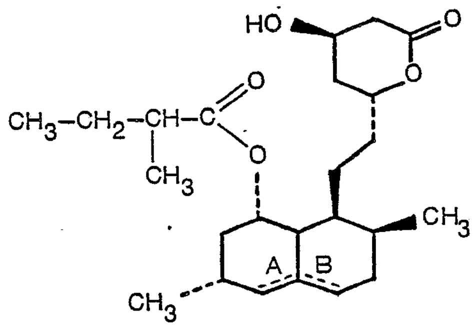

II

und deren freie Säureform

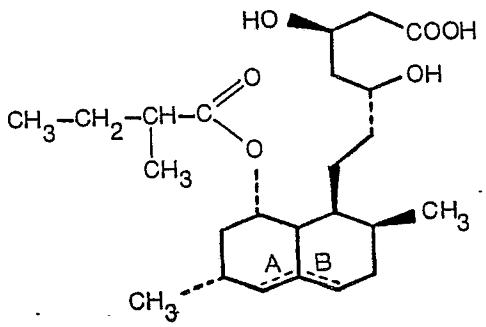

III

worin die strichlierten, mit A und B bezeichneten Linien fakultative Doppelbindungen darstellen, nicht mehr als eines der Symbole A und B eine Doppelbindung angibt, und, falls weder A noch B eine Doppelbindung angibt, der Perhydronaphthylring in der 4aα, 8aβ-Konfiguration vorliegt, sowie die pharmazeutisch annehmbaren Salze der genannten freien Säure und die Methyl-, Ethyl-, Propyl-, Butyl-, Isobutyl-, Amyl-, Isoamyl-, 2-Dimethylaminoethyl-, Benzyl-, Phenethyl- und 2-Acetamidoethylester.

2. Die Verbindung des Anspruchs 1, welche das Lacton der Formel II ist.

3. Die pharmazeutisch annehmbaren Salze der freien Säure der Formel III von Anspruch 1.

4. Die freie Säureform der Verbindung III von Anspruch 1.

5. Die Verbindungen des Anspruchs 2, in welchen A eine Doppelbindung ist.

6. Die Verbindung des Anspruchs 2, in welcher B eine Doppelbindung ist.

7. Die Verbindungen des Anspruchs 2, in welchen weder Anoch B eine Doppelbindung ist und das Ringsystem transkondensiert ist.

8. Ein Verfahren zur Herstellung der Verbindungen des Anspruchs 1, welches das Unterwerfen der Verbindung

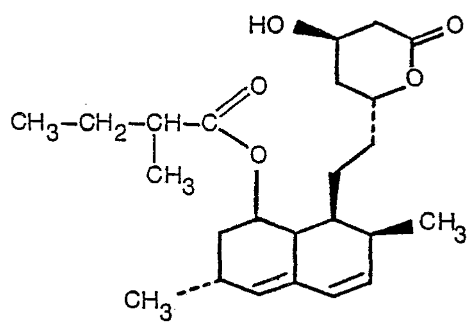

der Hydrierung entweder

a) über Tris(triphenylphosphonium)rhodiumchlorid unter Bildung einer Verbindung, in welcher B eine Doppelbindung ist, oder

b) über Palladium auf Calciumcarbonat unter Bildung einer Verbindung, in welcher A eine Doppelbindung ist, oder

c) über Platinoxid unter Bildung einer Verbindung, in welcher weder A noch B eine Doppelbindung ist, umfaßt.

9. Ein Verfahren zur Herstellung der Verbindung des Anspruchs 1, worin weder A noch B eine Doppelbindung ist, welches das Unterwerfen der Verbindung

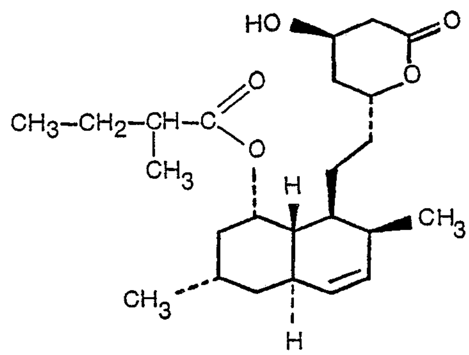

IV

der Hydrierung über Palladium auf Tierkohle umfaßt.

10. Eine antihypercholesterinämische, pharmazeutische Zusammensetzung, enthaltend einen pharmazeutischen Träger und eine wirksame Menge einer Verbindung der Formel II oder III des Anspruchs 1.

**Patentansprüche für den Vertragsstaat: AT**

1. Ein Verfahren zur Herstellung einer Verbindung der Formel:

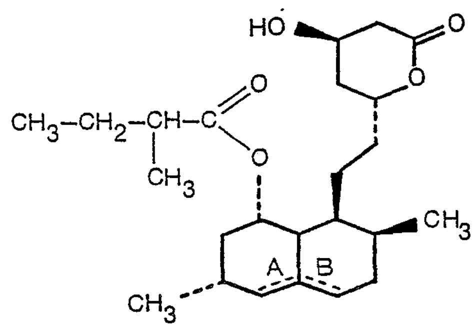

II

und deren freier Säureform

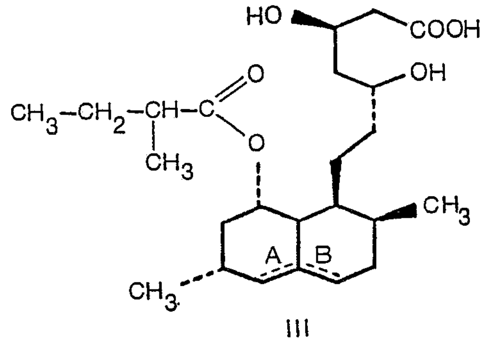

III

worin die strichlierten, mit A und B bezeichneten Linien fakultative Doppelbindungen darstellen, nicht mehr als eines der Symbole A und B eine Doppelbindung angibt, und, falls weder A noch B eine Doppelbindung angibt, der Perhydronaphthylring in der 4aα, 8aβ-Konfiguration vorliegt, sowie die pharmazeutisch annehmbaren Salze der genannten freien Säure und die Methyl-, Ethyl-, Propyl-, Butyl-, Isobutyl-, Amyl-, Isoamyl-, 2-Dimethylaminoethyl-, Benzyl-, Phenethyl- und 2-Acetamidoethylester, umfassend das Unterwerfen der Verbindung

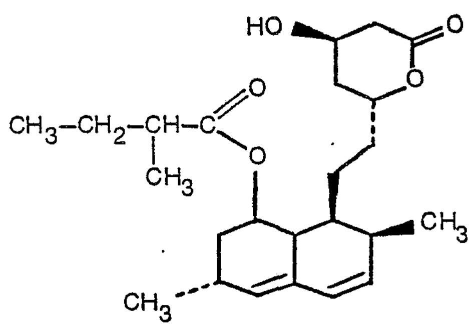

der Hydrierung entweder

a) über Tris(triphenylphosphonium)rhodiumchlorid unter Bildung einer Verbindung, in welcher B eine Doppelbindung ist, oder

b) über Palladium auf Calciumcarbonat unter Bildung einer Verbindung, in welcher A eine Doppelbindung ist, oder

c) über Platinoxid unter Bildung einer Verbindung, in welcher weder A noch B eine Doppelbindung ist, und, gewünschtenfalls, das Umsetzen der entstandenen Pyranonverbindung mit einer geeigneten Base, und, gewünschtenfalls, das Umsetzen des entstandenen Salzes des Säureverbindung mit einer anorganischen Säure, oder gewünschtenfalls, das Umsetzen der entstandenen Pyranonverbindung mit dem den oben erwähnten Estern entsprechenden Alkoxid.

2. Ein Verfahren zur Herstellung der Verbindung des Anspruchs 1 worin weder A noch B eine Doppelbindung ist, welches das Unterwerfen der Verbindung

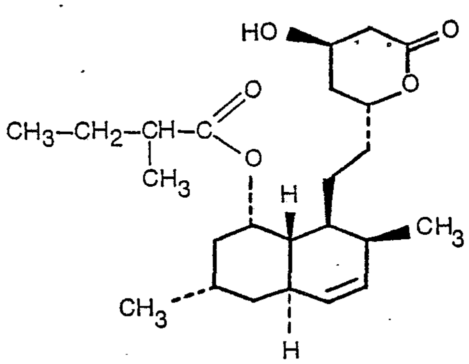

IV

der Hydrierung über Palladium auf Tierkohle umfaßt.

**Revendications pour les Etats Contractants: BE CH DE FR GB IT LI LU NL SE**

1. Composé de formule:

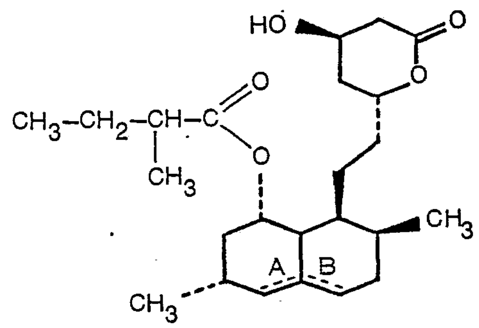

II

et sa forme acide libre

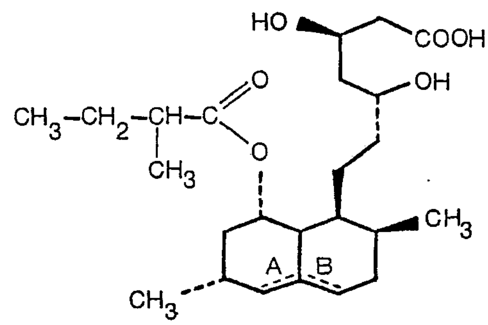

III

dans lesquels les lignes pointillées notées A et B représentent des doubles liaisons au choix, une au plus de A et de B étant une double liaison et, lorsque ni A ni B n'est une double liaison, le noyau perhydronaphtyle possède la configuration 4aα, 8aβ, ainsi que les sels pharmaceutiquement acceptables de cet acide libre et ses esters méthylique, éthylique, propylique, butylique, isobutylique, amylique, isoamylique, diméthyl-2 amino-éthylique, benzylique, phényléthylique et acétamido-2 éthylique.

2. Composé selon la revendication 1, caractérisé en ce qu'il est le lactone de formule II.

3. Sels pharmaceutiquement acceptables de l'acide libre de formule III selon la revendication 1.

4. Forme acide libre du composé III selon la revendication 1.

5. Composés selon la revendication 2, caractérisés en ce que A est une double liaison.

6. Composés selon la revendication 2, caractérisés en ce que B est une double liaison.

7. Composés selon la revendication 2, caractérisés en ce que ni A ni B n'est une double liaison et le système du noyau est fusionné en trans.

8. Procédé de production des composés selon la revendication 1, caractérisé en ce qu'il comprend la soumission du composé

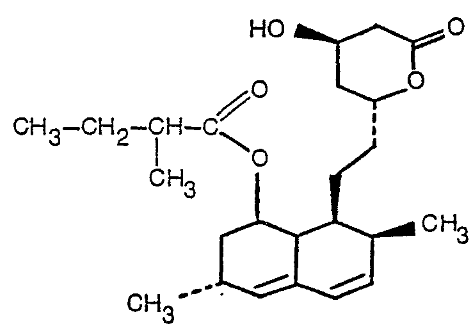

à une hydrogénation soit

a) sur chlorure de tris-(triphénylphosphonium)-rhodium pour produire un composé dans lequel B est une double liaison, ou

b) sur palladium sur carbonate de calcium pour produire un composé dans lequel A est une double liaison, ou

c) sur oxyde de platine pour produire un composé dans lequel ni A ni B n'est une double liaison.

9. Procédé de production d'un composé selon la revendication 1, dans lequel ni A ni B n'est une double liaison, caractérisé en ce qu'il comprend la soumission du composé

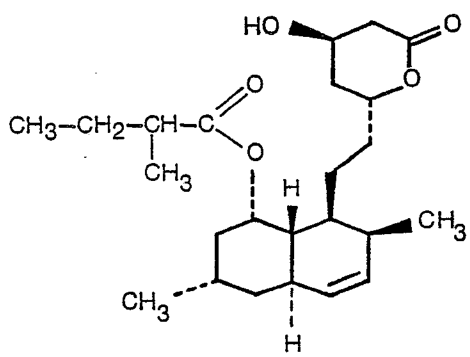

IV

à une hydrogénation sur palladium sur charbon.

10. Composition pharmaceutique antihypercholestérolémique contenant un véhicule pharmaceutique et une quantité efficace d'un composé de formule II ou III selon la revendication 1.

**Revendications pour l'Etat Contractant: AT**

1. Procédé de production d'un composé de formule

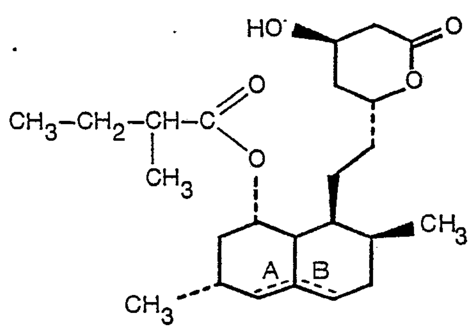

II

et sa forme acide libre

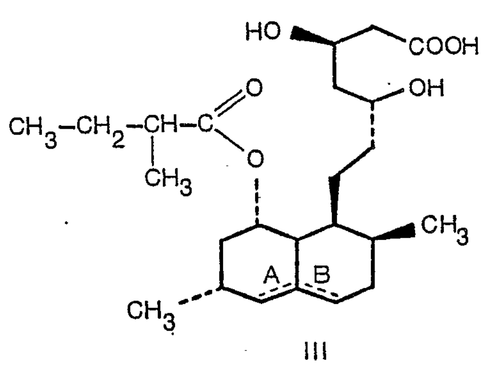

III

dans lesquels les lignes pointillées notées A et B représentent des doubles liaisons au choix, une au plus de A et de B étant une double liaison et, lorsque ni A ni B n'est une double liaison, le noyau perhydronaphtyle possède la configuration 4aα, 8aβ, ainsi que les sels pharmaceutiquement acceptables de cet acide libre et ses esters méthylique, éthylique, propylique, butylique, isobutylique, amylique, isoamylique, diméthyl-2 amino-éthylique, benzylique, phényléthylique et acétamido-2 éthylique, caractérisé en ce qu'il comprend la soumission du composé

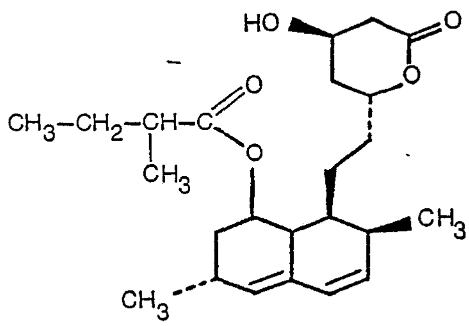

à une hydrogénation soit

a) sur chlorure de tris-(triphénylphosphonium)-rhodium pour produire un composé dans lequel B est une double liaison, ou

b) sur palladium sur carbonate de calcium pour produire un composé dans lequel A est une double liaison, ou

c) sur oxyde de platine pour produire un composé dans lequel ni A ni B n'est une double liaison et, éventuellement, la réaction du composé pyranone obtenu avec une base appropriée et, éventuellement, la réaction du sel du composé acide obtenu avec un acide minéral, ou, éventuellement, la réaction du composé pyranone obtenu avec l'alcoxyde correspondant aux esters mentionnés ci-dessus.

2. Procédé de production d'un composé selon la revendication 1, dans lequel ni A ni B n'est une double liaison, caractérisé en ce qu'il comprend la soumission du composé

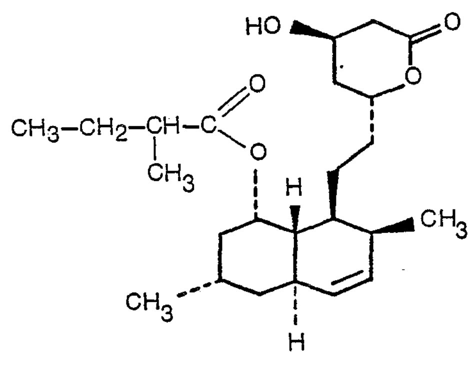

IV

à une hydrogénation sur palladium sur charbon.